# FASCICULE DE BREVET EUROPEEN

(11) **EP 1 418 293 B1**
(45) Date de publication et mention de la délivrance du brevet: **28.05.2008**
(21) Numéro de dépôt: 03292728.7
(22) Date de dépôt: 31.10.2003
(51) Int. Cl.: E04H 4/00

(54) **Systéme pour réaliser une cloison de retenue de liquide, telle une cloison de piscine, à partir de panneaux préfabriqués**
System zum Herstellen einer flüssigkeitszurückhaltenden Wand, z.B. Swimmbeckenwand, mit vorgefertigten Panelen
System for the construction of water retaining walls, such as swimming pool walls, with prefabricated panels

(30) Priorité: 08.11.2002 FR 0214078
(43) Date de publication de la demande: 12.05.2004
(73) Titulaire: Diffusion Equipements Loisirs, 35530 Brece (FR)
(72) Inventeur: Maupas, Alain, 56740 Locmariaquer (FR)
(74) Mandataire: Schwartz, Thierry J.

(56) Documents cités:
- FR-A- 2 735 806
- US-A- 3 986 310
- US-A- 4 974 266
- US-A- 5 155 872

## Description

L'invention concerne un système pour réaliser une cloison de retenue de liquide, telle une cloison de piscine, à partir de panneaux préfabriqués, ainsi qu'une piscine réalisée avec un tel système.

On connaît du document EP-0 799 952, un système d'assemblage de panneaux préfabriqués pour réaliser notamment une cloison de piscine, le système d'assemblage de deux panneaux consécutifs étant assuré par un profilé interne rigide qui est monté entre deux ailes adjacentes de deux panneaux consécutifs, et un profilé externe rigide ou semi-rigide qui vient coiffer les deux ailes, le profilé externe étant généralement monté en premier, alors que le profilé interne est engagé ensuite à force ou avec un faible jeu.

Le but de l'invention est de perfectionner le système d'assemblage tel que décrit dans le document précité, de manière à pouvoir permettre notamment la réalisation d'une cloison de retenue d'eau à partir de panneaux préfabriqués d'un seul type et ce, quelle que soit la forme du contour rectiligne et/ou courbe du contour de la cloison.

A cet effet, l'invention propose un système pour réaliser une cloison de retenue de liquide, telle une cloison de piscine, à partir de panneaux préfabriqués, ce système comprenant au moins des moyens d'assemblage de deux panneaux consécutifs disposés verticalement, une ceinture basse qui supporte les panneaux et une ceinture haute qui est rapportée sur les panneaux, caractérisée en ce que les moyens d'assemblage ont pour fonction de maintenir deux panneaux assemblés l'un à l'autre tout en permettant un débattement angulaire de l'un par rapport à l'autre autour d'un axe vertical, et en ce qu'il comprend également des moyens de conformation d'angle pour assurer une orientation angulaire déterminée entre au moins deux panneaux consécutifs en fonction du contour de la cloison à réaliser, et des moyens de rigidification des panneaux une fois assemblés les uns aux autres, selon la revendication 1.

Avantageusement, la cloison peut être réalisée à partir de panneaux plans qui peuvent indifféremment montés dans une partie rectiligne ou courbe du contour de la cloison à réaliser, sans qu'il soit nécessaire de réaliser des panneaux cintrés dans une partie courbe.

Selon un exemple de réalisation, tous les panneaux de la cloison sont identiques.

D'une manière générale, la ceinture basse qui supporte les panneaux est constituée par un ensemble de profilés à section droite en U qui forment des goulottes, chaque profilé s'étendant sur une longueur au moins égale à celle d'un panneau, et la ceinture haute qui est rapportée sur les panneaux est constituée par un ensemble de bandeaux associé à un ensemble de profilés à section droite en U qui forment des goulottes, chaque bandeau et son profilé associé s'étendant sur une longueur au moins égale à celle d'un panneau, les ceintures basse et haute étant reliées l'une à l'autre par l'un des profilés des moyens d'assemblage.

Les moyens de conformation d'angle entre deux panneaux consécutifs sont logés dans la ceinture basse et/ou la ceinture haute, en particulier dans les goulottes des profilés.

Par ailleurs, des moyens sont prévus pour assurer un nivellement en hauteur des panneaux, ainsi que des moyens de rigidification des ceintures basse et haute pour figer le contour de la cloison une fois les panneaux assemblés.

Selon un avantage important de l'invention, il est possible de réaliser des cloisons de piscine ayant un contour quelconque avec des panneaux qui peuvent être d'un seul type, ce qui simplifie considérablement les problèmes de fabrication, de maintenance et de stockage des panneaux.

Par ailleurs, les faibles dimensions des panneaux facilitent leur manipulation et leur transport.

A titre d'exemple, chaque panneau est de forme sensiblement rectangulaire, s'étend sur une hauteur de l'ordre de 1,10m et sur une longueur de l'ordre de 50cm, chaque panneau étant réalisé à partir d'une feuille métallique ou tôle ayant une épaisseur de l'ordre de 1,5 à 2mm, ce qui est avantageux du point de vue du coût de fabrication.

D'autres avantages, caractéristiques et détails de l'invention ressortiront du complément de description qui va suivre en référence à des dessins annexés, donnés uniquement à titre d'exemple et dans lesquels :
- la figure 1 est une vue en perspective pour illustrer deux panneaux consécutifs de la cloison de piscine avant leur assemblage, ainsi que deux vues de détails I et II ;
- la figure 2 est une vue en coupe pour illustrer les moyens d'assemblage des deux panneaux illustrés à la figure 1 ;
- la figure 3 est une vue en perspective de l'un des moyens d'assemblage illustrés à la figure 2 ;
- la figure 4 est une vue en perspective partielle pour illustrer la ceinture basse de la cloison qui supporte les panneaux ;
- les figures 5 et 6 sont des vues en coupe schématiques pour illustrer la ceinture haute de la cloison qui est rapportée sur les panneaux ; et
- la figure 7 est une vue en perspective partielle pour illustrer une partie seulement de la ceinture haute de la cloison.

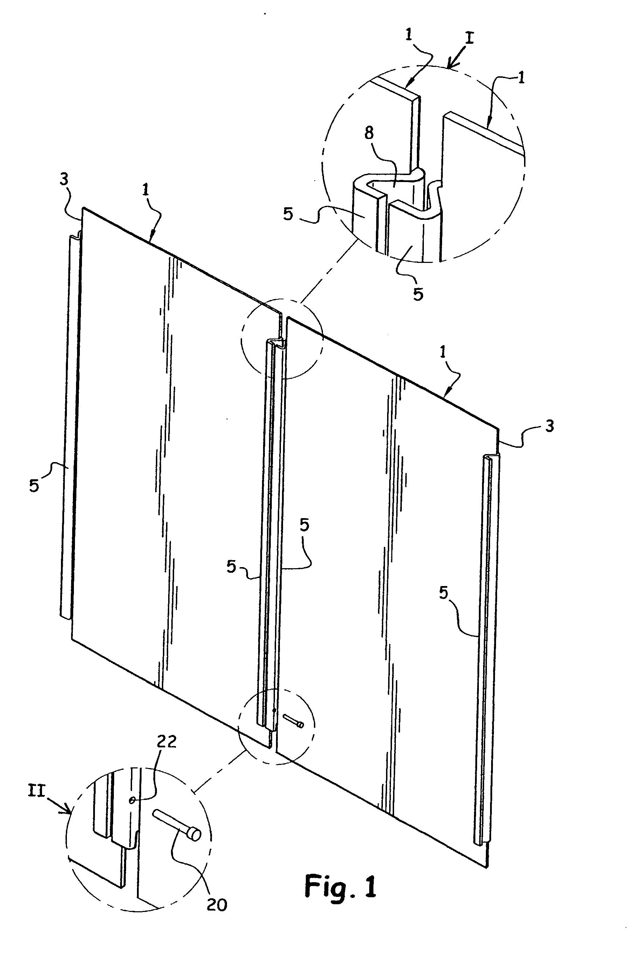

Soit à réaliser une cloison de retenue d'eau, une cloison de piscine à contour fermé par exemple, à partir de panneaux préfabriqués 1 tels que ceux illustrés à la figure 1. Chaque panneau est plan et présente une forme rectangulaire avec deux bords latéraux 3 qui délimitent la hauteur du panneau 1. Chaque panneau 1 est usiné de manière à présenter une aile repliée 5 sur chacun de ses bords latéraux 3, ces deux ailes 5 étant repliées d'un même côté du panneau 1. Selon l'exemple illustré à la figure 1, les deux ailes 5 de chaque panneau 1 ne s'étendent que sur une partie de la hauteur du panneau 1. Les ailes 5 de chaque panneau 1 sont conformées de manière à ce que les deux ailes adjacentes 5 de deux panneaux consécutifs 1 délimitent entre elles une gorge ou rainure verticale 8 à section droite sensiblement circulaire, comme cela ressort du détail I de la figure 1.

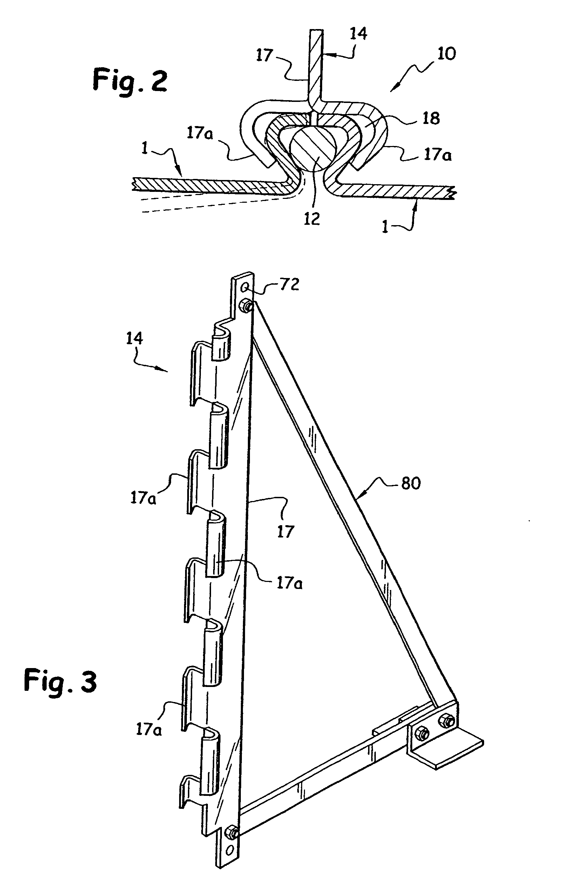

Deux panneaux consécutifs 1 sont raccordés l'un à l'autre par des moyens d'assemblage 10 aptes à coopérer avec les deux ailes adjacentes 5 de deux panneaux consécutifs 1. Selon l'exemple de réalisation illustré à la figure 2, les moyens d'assemblage 10 comprennent deux profilés rigides ou semi-rigides respectivement interne 12 et externe 14.

Le premier profilé interne 12 sous la forme d'une tige vient se loger à l'intérieur de la rainure 8 en étant introduite librement par une extrémité de la rainure 8. Le second profilé 14 est rapporté de manière à venir coiffer les deux ailes adjacentes 5 de deux panneaux consécutifs 1. Selon un exemple de réalisation illustré à la figure 3, le second profilé 14 est en une seule pièce et se présente sous la forme d'une plaque rectangulaire 17 dont un bord longitudinal présente des ailes alternées 17a qui délimitent sur une partie de la hauteur de la plaque 17 une rainure 18 discontinue apte à venir entourer les deux ailes adjacentes 5 de deux panneaux consécutifs 1. Le second profilé 14 est rapporté verticalement le long des ailes 5. L'ordre de montage des deux profilés 12 et 14 des moyens d'assemblage 10 est indifférent.

En effet, une fois les moyens d'assemblage rapportés sur deux panneaux consécutifs 1, il y a la possibilité d'un débattement angulaire d'un panneau par rapport à l'autre autour d'un axe vertical qui est matérialisé par la tige formant le premier profilé 12 des moyens d'assemblage 10, comme cela est schématiquement illustré en traits pointillés sur la figure 2. Un débattement angulaire est formé entre au moins deux panneaux consécutifs 1 lorsque l'on veut réaliser une partie courbe de la cloison.

En se rapportant à nouveau à la figure 1, en particulier au détail II, des moyens 20 sous la forme d'un pion par exemple vient s'engager entre les deux ailes adjacentes 5 de deux panneaux consécutifs 1 pour assurer un nivellement de la hauteur des panneaux 1. Le pion est par exemple introduit dans deux trous 22 percés dans les ailes 5 des panneaux 1. Ces trous 22 sont par exemple situés à la partie inférieure des ailes 5 pour ne pas gêner le montage du premier profilé 12 des moyens d'assemblage 10.

Le système pour réaliser la cloison de piscine comprend également une ceinture basse 25 sur laquelle repose les panneaux 1 et une ceinture haute 27 qui est rapporté sur les panneaux 1.

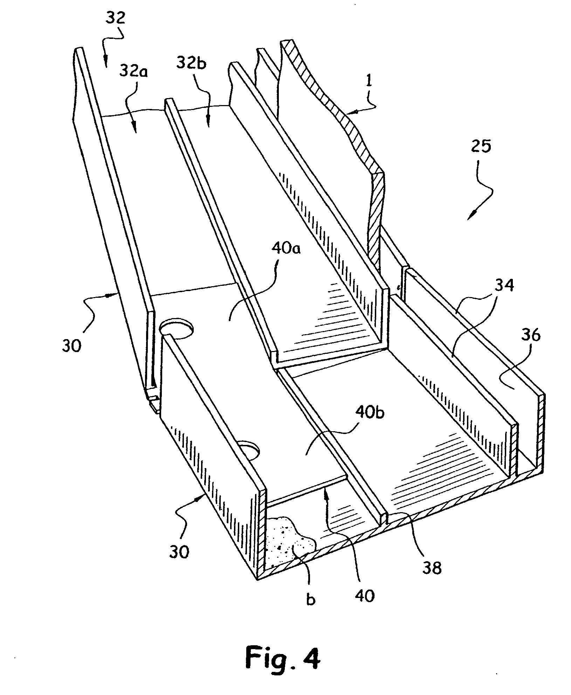

La ceinture basse 25 telle qu'illustrée partiellement sur la figure 4 est constituée par un ensemble de profilés rectilignes 30 à section droite en U qui forment chacun une goulotte 32. Un bord de la chaque profilé 30 présente une double paroi 34 qui délimite une rainure 36 dans laquelle vient se loger la partie basse d'au moins un panneau 1. Chaque profilé 30 s'étend sur une longueur au moins égale à celle d'un panneau 1 qu'il supporte. Dans sa partie centrale, chaque profilé 30 présente une nervure 38 qui scinde la goulotte 32 en deux parties 32a et 32b. La partie 32a de la goulotte 32 qui est opposée aux panneaux 1 est destinée à recevoir des moyens de conformation d'angle 40 entre deux panneaux successifs 1. Chaque moyen de conformation d'angle 40 est formé par une pièce plane qui présente deux ailes 40a et 40b qui forment un certain angle entre elles de manière à orienter deux panneaux successifs 1 l'un par rapport à l'autre suivant l'angle souhaité, les deux ailes 40a et 40b venant respectivement se loger dans les deux goulottes adjacentes 32 de deux panneaux consécutifs 1.

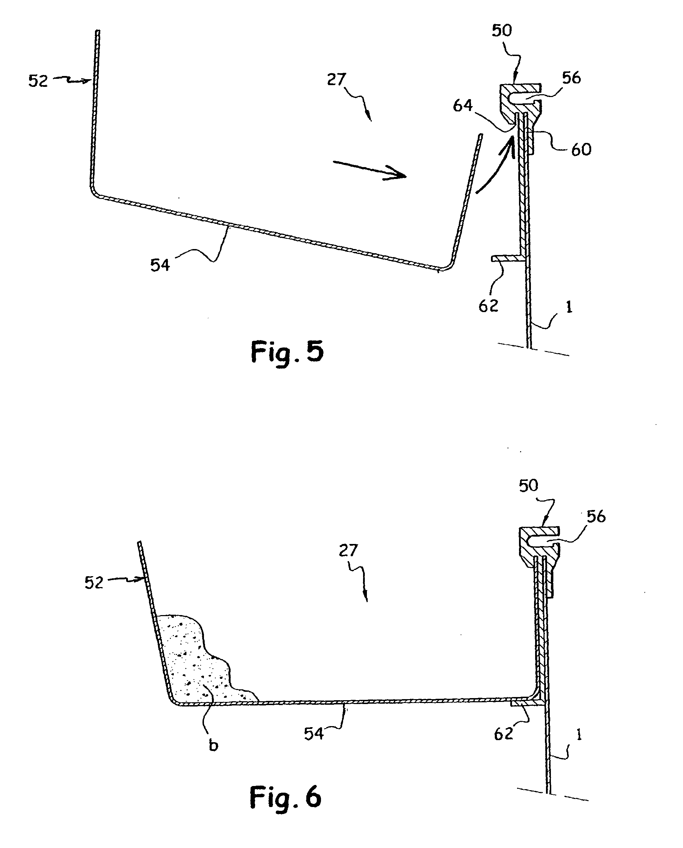

La ceinture haute 27 telle que schématiquement illustrée sur les figures 5 et 6 comprend un ensemble de bandeaux 50 qui sont rapportés sur les panneaux 1, et un ensemble de profilés 52 à section droite en U qui forment chacun une goulotte 54. Chaque bandeau 50 et chaque profilé associé 52 s'étend sur une longueur au moins égale à celle d'un panneau 1.

Chaque bandeau 50 présente une encoche latérale 56 dans laquelle vient se monter la bâche ou "liner" de la piscine. Chaque bandeau 50 présente une double paroi qui délimite une rainure 60 dans laquelle vient s'engager la partie haute des panneaux 1. A sa partie inférieure, chaque bandeau 50 présente un rebord 62 sensiblement à 90° qui sert de surface d'appui pour la goulotte 54 d'un profilé 52, alors qu'un bord supérieur de la goulotte 54 vient s'engager dans une rainure 64 du bandeau 50. Le montage d'une goulotte 54 dans son bandeau 50 associé est illustré sur les figures 5 et 6, ce montage se faisant par emmanchement pour éviter d'avoir recours à des moyens de fixation supplémentaires.

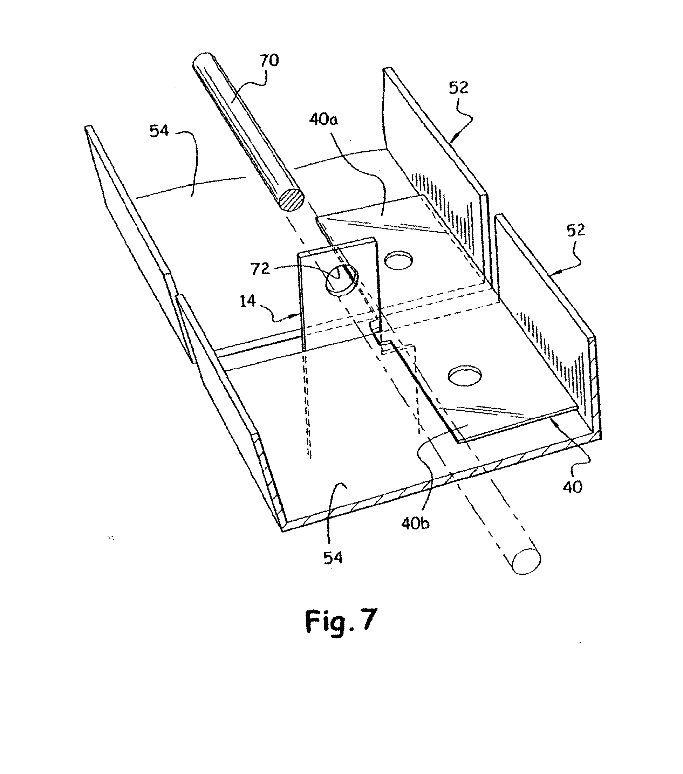

Des moyens de conformation d'angle 40, similaires à ceux de la ceinture basse 25, sont positionnés dans le fond de deux goulottes adjacentes 54 de deux panneaux consécutifs 1 pour réaliser une partie courbe. Dans l'exemple illustré sur la figure 7, le moyen de conformation d'angle 40 a ses deux ailes 40a et 40b dans le prolongement l'une de l'autre, c'est-à-dire que la conformation de l'angle est de 180°, et les deux panneaux consécutifs 1 sont en fait alignés l'un avec l'autre. Dans ce cas, le moyen de conformation d'angle est un simple élément rectiligne de rigidification. Par contre, lorsque deux panneaux consécutifs 1 ne sont pas alignés l'un avec l'autre, deux moyens de conformation d'angle 40 sont montés en regard l'un de l'autre dans deux goulottes adjacentes 32 de la ceinture basse 25 et dans deux goulottes adjacentes 54 de la ceinture haute 27, respectivement.

Une fois l'ensemble des panneaux 1 assemblés les uns aux autres pour réaliser une forme de contour rectiligne et/ou courbe, on fige cette forme par des moyens de rigidification ou de renfort qui vont notamment fixés rigidement l'une à l'autre les ceintures basse 25 et haute 27. Plus précisément, les ceintures basse 25 et haute 27 de la cloison de piscine sont reliées l'une à l'autre par l'intermédiaire des seconds profilés 14 des moyens d'assemblage 10 de deux panneaux successifs 1, les deux extrémités de chaque second profilé 14 des moyens d'assemblage 10 faisant respectivement saillie entre deux goulottes adjacentes 32 de la ceinture basse 25 d'une part, et entre deux goulottes adjacentes 54 de la ceinture haute 27 d'autre part. Ensuite, des moyens de rigidification sont mis en place et, à titre d'exemple, ils sont constitués par du béton b qui est coulé dans les goulottes 32 et 54 des ceintures basse 25 et haute 27 (figures 4 et 6). Avantageusement, on peut prévoir une armature métallique 70 à l'intérieur des goulottes 54 de la ceinture haute 27, cette armature 70 pouvant être sous la forme d'une tige métallique qui entoure la ceinture haute 27 en passant au travers d'une ouverture 72 percée à la partie supérieure des seconds profilés 14 des moyens d'assemblage 10. Ainsi, le béton assure la liaison rigide entre les ceintures basse 25 et haute 27 par l'intermédiaire du second profilé 14.

En se reportant à nouveau à la figure 3, chaque second profilé 14 des moyens d'assemblage 10 peut être équipé d'une jambe de renfort 80 qui vient se fixer aux deux extrémités de chaque profilé 14.

Le montage de la cloison s'effectue globalement en deux étapes principales. La première étape consiste à assembler les panneaux entre eux de manière à obtenir la forme du contour rectiligne et/ou courbe de la cloison. La seconde étape consiste à rigidifier cette forme en coulant du béton dans les ceintures basse et haute selon l'exemple décrit précédemment.

Ainsi, la cloison de la piscine peut être avantageusement réalisée à partie de panneaux qui peuvent être indifféremment montés dans une partie rectiligne ou courbe du contour de la cloison, des panneaux cintrés n'étant pas nécessaires pour réaliser des parties courbes.

En variante du mode de réalisation qui a été décrit précédemment, le second profilé 14 des moyens d'assemblage 10 pourrait être réalisé en deux parties rapportées l'une contre l'autre, puis fixées ensemble par tout moyen approprié. Les moyens de rigidification ou de renfort des ceintures basse 25 et haute 27 pourraient être réalisés par des moyens autres que du béton qui n'a été donné qu'à titre d'exemple.

Par ailleurs, dans les parties rectilignes de la cloison, on pourrait réaliser les profilés 30 de la ceinture basse 25 sur une longueur supérieure à celle d'un panneau 1 de manière à pouvoir supporter plusieurs panneaux. Il pourrait en être de même pour les bandeaux 50 et les profilés associés de la ceinture haute 27.

D'une manière générale, les panneaux 1 sont avantageusement réalisés à partir de feuilles métalliques ou tôles d'une épaisseur de l'ordre de 1,5 à 2mm, mais on pourrait envisager un autre matériau du type plastique.

Enfin, chaque panneau 1 pourrait être réalisé sous la forme d'un caisson avec deux tôles respectivement interne et externe parallèles entre elles, et qui seraient assemblées l'une à l'autre au moyen d'une ceinture basse et d'une ceinture haute. Une forme de caisson des panneaux permettraient d'y loger des équipements tels q'une pompe, des filtres, ..., nécessaires au fonctionnement et à l'entretien d'une piscine.

## Revendications

1. Système pour réaliser une cloison de retenue de liquide, telle une cloison de piscine, à partir de panneaux préfabriqués (1), ce système comprenant au moins des moyens d'assemblage (10) de deux panneaux consécutifs (1) disposés verticalement, une ceinture basse (25) qui supporte les panneaux (1) et une ceinture haute (27) qui est rapportée sur les panneaux (1), les moyens d'assemblage (10) ayant pour fonction de maintenir deux panneaux assemblés (1) l'un à l'autre tout en permettant un débattement angulaire de l'un par rapport à l'autre autour d'un axe vertical, et comprenant également des moyens de conformation d'angle (40) pour assurer une orientation angulaire déterminée entre au moins deux panneaux consécutifs (1) en fonction du contour de la cloison à réaliser, et des moyens de rigidification des panneaux (1) une fois assemblés les uns aux autres, **caractérisé en ce que** la cloison est réalisée à partir de panneaux plans (1) qui sont indifféremment montés dans une partie rectiligne ou courbe du contour de la cloison à réaliser, et **en ce que** chaque moyen de conformation d'angle (40) est constitué par une pièce présentant deux branches (40a, 40b) formant un angle déterminé entre elles.

2. Système selon la revendication 1, **caractérisé en ce que** tous les panneaux (1) sont identiques.

3. Système selon la revendication 1 ou 2, **caractérisé en ce qu'**il comprend des moyens (20) pour assurer un nivellement de la hauteur de deux panneaux consécutifs (1).

4. Système selon l'une des revendications précédentes, **caractérisé en ce que** les ceintures basse (25) et haute (27) du système sont reliées rigidement l'une à l'autre par une partie des moyens d'assemblage (10).

5. Système selon l'une des revendications précédentes, **caractérisé en ce que** les moyens de conformation d'angle (40) entre au moins deux panneaux consécutifs (1) sont situés dans la ceinture basse (25) et/ou la ceinture haute (27) du système.

6. Système selon l'une des revendications précédentes, **caractérisé en ce que** la ceinture basse (25) qui supporte les panneaux (1) est constituée par une pluralité de profilés (30), chaque profilé (30) s'étendant au moins sur une longueur correspondant au moins à celle d'un panneau (1) qu'il supporte.

7. Système selon la revendication 6, **caractérisé en ce que** chaque profilé de la ceinture basse (25) est rectiligne et forme une goulotte (32) à section droite sensiblement en U avec une paroi double (34) qui s'étend sur l'un de ses côtés longitudinaux pour délimiter une rainure (36) dans laquelle s'engage la partie basse d'au moins un panneau (1).

8. Système selon la revendication 7, **caractérisé en ce que** des moyens de conformation d'angle (40) sont montés dans deux goulottes (32) adjacentes formées par deux profilés consécutifs (30) de la ceinture basse (25).

9. Système selon la revendication 7 ou 8, **caractérisé en ce que** chaque profilé (30) de la ceinture basse (25) est rigidifié par des moyens de renfort.

10. Système selon la revendication 9, **caractérisé en ce que** les moyens de renfort sont constitués par du béton (b) qui est coulé dans la goulotte (32) formée par chaque profilé (30) de la ceinture basse (25).

11. Système selon l'une des revendications précédentes, **caractérisé en ce que** la ceinture haute (27) qui est rapportée sur les panneaux (1) est constituée par un ensemble de bandeaux (50) et un ensemble de profilés (52) respectivement fixés aux bandeaux (50), chaque bandeau (50) et chaque profilé (52) s'étendant sur une longueur au moins égale à celle d'un panneau (1) sur lequel ils sont rapportés.

12. Système selon la revendication 11, **caractérisé en ce que** chaque profilé (52) associé à un bandeau (50) est fixé par emmanchement dans ledit profilé (52).

13. Système selon la revendication 12, **caractérisé en ce que** chaque profilé (52) forme une goulotte (54) à section droite sensiblement en U, et **en ce que** des moyens de conformation d'angle (40) sont montés dans deux goulottes (54) adjacentes formées par deux profilés consécutifs (52) de la ceinture haute (27).

14. Système selon la revendication 13, **caractérisé en ce que** chaque profilé (52) de la ceinture haute (27) est rigidifié par des moyens de renfort.

15. Système selon la revendication 14, **caractérisé en ce que** les moyens de renfort sont constitués par du béton (b) qui est coulé dans la goulotte (54) formée par chaque profilé (52) de la ceinture haute (27).

16. Système selon l'une des revendications précédentes, **caractérisé en ce que** chaque panneau (1) monté en position verticale présente deux bords latéraux verticaux (3), chaque bord latéral (3) présentant sur tout ou partie de sa hauteur une aile (5), les deux ailes (5) d'un panneau (1) étant repliées d'un même côté de celui-ci, et **en ce que** les moyens d'assemblage (10) entre deux panneaux consécutifs (1) comprennent au moins un premier profilé interne (12) monté entre les deux ailes adjacentes (5) de deux panneaux consécutifs (1), et au moins un second profilé externe (14) qui coiffe les deux ailes adjacentes (5) desdits panneaux (1) et qui pénètre à l'intérieur des ceintures basse (25) et haute (27).

17. Système selon la revendication 16, **caractérisé en ce qu**'il comprend des moyens (20) pour assurer un nivellement de la hauteur de deux panneaux consécutifs (1).

18. Système selon la revendication 17, **caractérisé en ce que** les moyens (20) sont constitués par un pion qui s'engage dans deux trous (22) percés à la partie inférieure des ailes (5) des panneaux (1).

19. Système selon l'une des revendications précédentes, **caractérisé en ce que** chaque panneau (1) est réalisé à partir d'une feuille métallique d'une épaisseur de l'ordre de 1,5 à 2mm.

20. Système selon la revendication 19, **caractérisé en ce que** chaque panneau (1) est réalisé sous la forme d'un caisson formé à partir de deux feuilles métalliques reliées entre elles par les ceintures basse (25) et haute (27).

21. Cloison de retenue d'eau, en particulier une cloison de piscine, **caractérisée en ce qu'**elle est réalisée à partir d'un système tel que défini par l'une des revendications précédentes.

## Claims

1. A system for making a liquid-retaining wall, such as a swimming pool wall, from prefabricated panels (1), the system comprising at least assembly means (10) for assembling together two consecutive vertically-disposed panels (1), a low belt (25) which supports the panels (1), and a high belt (27) which is fitted to the panels (1), the assembly means (10) having the function of holding together two assembled-together panels (1) while allowing one of the panels to move angularly relative to the other about a vertical axis, and also including angle-determining means (40) for ensuring that at least two consecutive panels (1) are at a determined angular orientation relative to each other as a function of the outline of the wall to be made, and further including stiffening means for stiffening the panels (1) once they have been assembled to one another, **characterized in that** the wall is made up of plane panels (1) which are equally mounted in a rectilinear or curved portion of the outline of the wall to be made, and **in that** each angle-determining means (40) is constituted by a part presenting two arms (40a, 40b) that form a determined angle relative to each other.

2. A system according to claim 1, **characterized in that** all the panels (1) are identical.

3. A system according to claim 1 or claim 2, **characterized in that** it includes means (20) for leveling the heights of two consecutive panels (1).

4. A system according to any preceding claim, **characterized in that** the low and high belts (25, 27) of the system are rigidly connected to each other by a portion of the assembly means (10).

5. A system according to any preceding claim, **characterized in that** the angle-determining means (40) for shaping the angle between at least two consecutive panels (1) are situated in the low belt (5) and/or in the high belt (27) of the system.

6. A system according to any preceding claim, **characterized in that** the low belt (25) which supports the panels (1) is constituted by a plurality of section members (30), each section member (30) extending over a length that is at least as long as the length of a panel
(1) supported thereby.

7. A system according to claim 6, **characterized in that** each section member of the low belt (25) is rectilinear and forms a substantially channel section gutter (32) with a double wall (34) extending along one of its longitudinal sides to define a slot (36) in which the bottom portion of at least one panel (1) is engaged.

8. A system according to claim 7, **characterized in that** the angle-determining means (40) are mounted in two adjacent gutters (32) formed by two consecutive section members (30) of the low belt (25).

9. A system according to claim 7 or claim 8, **characterized in that** each section member (30) of the low belt (25) is stiffened by reinforcing means.

10. A system according to claim 9, **characterized in that** the reinforcing means are constituted by concrete (b) which is cast into the gutter (32) formed by each of the section members (30) of the low belt (25).

11. A system according to any preceding claim, **characterized in that** the high belt (27) which is fitted to the panels (1) is constituted by a set of strips (50) and by a set of section members (52) fixed respectively to the strips (50), each strip (50) and each section member (52) extending over a length that is at least as long as the length of a panel (1) on which they are fitted.

12. A system according to claim 11, **characterized in that** each section member (52) associated with a strip (50) is fixed by being engaged in said section member (52).

13. A system according to claim 12, **characterized in that** each section member (52) forms a substantially channel section gutter (54), and **in that** angle-determining means (40) are mounted in two adjacent gutters (54) formed by two consecutive section members (52) of the high belt (27).

14. A system according to claim 13, **characterized in that** each section member (52) of the high belt (27) is stiffened by reinforcing means.

15. A system according to claim 14, **characterized in that** the reinforcing means are constituted by concrete (b) which is cast into the gutter (54) formed by each section member (52) of the high belt (27).

16. A system according to any preceding claim, **characterized in that** each panel (1) mounted in a vertical position presents two vertical lateral edges (3), each lateral edge (3) presenting over all or part of its height a folded flange (5), the two flanges (5) of a panel (1) being folded towards the same side of the panel, and **in that** the assembly means (10) between two consecutive panels (1) comprise at least an internal, first section member (12) mounted between the two adjacent flanges (5) of two consecutive panels (1), and at least an external, second section member (14) fitted over the two adjacent flanges (5) of said panels (1) and which penetrate into the insides of the low and high belts (25, 27).

17. A system according to claim 16, **characterized in that** it includes leveling means (20) for leveling the heights of two consecutive panels (1).

18. A system according to claim 17, **characterized in that** the leveling means (20) are constituted by a pin which is engaged in two holes (22) pierced in the bottom portions of the flanges (5) of the panels (1).

19. A system according to any preceding claim, **characterized in that** each panel (1) is made from a metal sheet that is about 1.5 mm to 2 mm thick.

20. A system according to claim 19, **characterized in that** each panel (1) is made in the form of a box section built up from two metal sheets interconnected by the low and high belts (25, 27).

21. A water-retaining wall, in particular a swimming pool wall, **characterized in that** it is made using a system as defined in any preceding claim.

## Patentansprüche

1. System zur Herstellung einer Wand zum Zurückhalten von Flüssigkeit wie einer Schwimmbeckenwand ausgehend von vorgefertigten Platten (1), wobei das System zumindest Mittel zum Zusammenbauen (10) von zwei aufeinander folgenden Platten (1), welche vertikal angeordnet sind, einen unteren Gurt (25), welcher die Platten (1) trägt, und einen oberen Gurt (27), welcher auf den Platten (1) angepasst ist, umfasst, wobei die Mittel zum Zusammenbauen (10) die Funktion haben, zwei zusammengebaute Platten (1) nebeneinander zu halten, wobei sie einen winkelmäßigen Federweg der zwei Platten um eine vertikale Achse zueinander ermöglichen, und zudem Winkelgestaltungsmittel (4), um eine vorbestimmte Winkelausrichtung zwischen mindestens zwei aufeinander folgenden Platten (1) in Abhängigkeit von der Kontur der zu fertigenden Wand sicherzustellen, und Mittel zur Versteifung der einmal miteinander zusammengebauten Platten (1) umfassen, **dadurch gekennzeichnet, dass** die Wand ausgehend von planen Platten (1) gefertigt wird, welche gleichartig in einem geradlinigen oder gekrümmten Abschnitt der Kontur der zu fertigenden Wand angebracht sind, und dass jedes Winkelgestaltungsmittel (40) durch ein Teil gebildet ist, welches zwei Zweige (40a, 40b) aufweist, welche einen vorbestimmten Winkel zwischen sich bilden.

2. System nach Anspruch 1, **dadurch gekennzeichnet, dass** alle Platten (1) identisch sind.

3. System nach Anspruch 1 oder 2, **dadurch gekennzeichnet, dass** es Mittel (20) zum Sicherstellen einer Nivellierung der Höhe zweier aufeinanderfolgender Platten (1) umfasst.

4. System nach einem der vorhergehenden Ansprüche, **dadurch gekennzeichnet, dass** der untere (25) und obere (27) Gurt des Systems in steifer Weise miteinander durch einen Teil der Mittel zum Zusammenbauen (10) verbunden sind.

5. System nach einem der vorhergehenden Ansprüche, **dadurch gekennzeichnet, dass** die Winkelgestaltungsmittel (40) zwischen mindestens zwei aufeinander folgenden Platten (1) in dem unteren Gurt (25) und/oder dem oberen Gurt (27) des Systems angeordnet sind.

6. System nach einem der vorhergehenden Ansprüche, **dadurch gekennzeichnet, dass** der untere Gurt (25), welcher die Platten (1) trägt, durch eine Vielzahl von Profilen (30) gebildet ist, wobei sich jedes Profil (30) zumindest über eine Länge erstreckt, welche zumindest derjenigen einer Platte (1), welche es trägt, entspricht.

7. System nach Anspruch 6, **dadurch gekennzeichnet, dass** jedes Profil des unteren Gurts (25) geradlinig ist und eine Rinne (32) mit im Wesentlichen U-förmigen Querschnitt mit einer doppelten Wand (34), welche sich entlang einer seiner longitudinalen Seiten erstreckt, um eine Nut (36) zu definieren, mit welcher der untere Teil mindestens einer Platte (1) in Eingriff gelangt, bildet.

8. System gemäß Anspruch 7, **dadurch gekennzeichnet, dass** die Winkelgestaltungsmittel (40) in zwei benachbarten Rinnen (32) angebracht sind, welche durch zwei aufeinander folgende Profile (30) des unteren Gurtes (25) gebildet werden.

9. System gemäß Anspruch 7 oder 8, **dadurch gekennzeichnet, dass** jedes Profil (30) des unteren Gurtes (25) durch Verstärkungsmittel versteift ist.

10. System gemäß Anspruch 9, **dadurch gekennzeichnet, dass** die Verstärkungsmittel durch Beton (b) gebildet sind, welcher in die durch jedes Profil (30) des unteren Gurtes (25) gebildeten Rinne (32) geflossen ist.

11. System gemäß einem der vorhergehenden Ansprüche, **dadurch gekennzeichnet, dass** der obere Gurt (27), welcher auf die Platten (1) angepasst ist, durch eine Anordnung von Blenden (50) und eine Anordnung von Profilen (52), welche jeweils an den Blenden (50) befestigt sind, gebildet ist, wobei jede Blende (50) und jedes Profil (52) sich über eine Länge erstreckt, welche zumindest gleich derjenigen der Platte (1) ist, auf welche sie angepasst sind.

12. System gemäß Anspruch 11, **dadurch gekennzeichnet, dass** jedes einer Blende (50) zugeordnete Profil (52) durch Einpressen in dem Profil (52) befestigt ist.

13. System gemäß Anspruch 12, **dadurch gekennzeichnet, dass** jedes Profil (52) eine Rinne (54) mit im Wesentlichen U-förmigem Querschnitt bildet, und dass die Winkelgestaltungsmittel (40) in zwei benachbarten Rinnen (54) angebracht sind, welche durch zwei aufeinander folgende Profile (52) des oberen Gurtes (27) gebildet sind.

14. System gemäß Anspruch 13, **dadurch gekennzeichnet, dass** jedes Profil (52) des oberen Gurtes (27) durch Verstärkungsmittel versteift ist.

15. System gemäß Anspruch 14, **dadurch gekennzeichnet, dass** die Verstärkungsmittel durch Beton (b) gebildet sind, welcher in die durch jedes Profil (52) des oberen Gurtes (27) gebildeten Rinne (54) geflossen ist.

16. System gemäß einem der vorhergehenden Ansprüche, **dadurch gekennzeichnet, dass** jede in vertikaler Position angebrachte Platte (1) zwei laterale vertikale Ränder (3) aufweist, wobei jede laterale Kante (3) über ihre ganze oder einen Teil ihrer Höhe einen Flügel (5) aufweist, wobei die zwei Flügel (5) einer Platte (1) auf einer selben Seite derselben zusammengefaltet sind, und dass die Mittel zum Zusammenbauen (10) zwischen zwei aufeinander folgenden Platten (1) zumindest ein erstes internes Profil (12), welches zwischen den zwei benachbarten Flügel (5) zweier aufeinander folgenden Platten (1) angebracht ist, und zumindest ein zweites externes Profil (14), welches auf den zwei benachbarten Flügeln (5) der Platten (1) aufsetzt und welches in das Innere des unteren (25) und oberen (27) Gurtes hineinragt, aufweisen.

17. System gemäß Anspruch 16, **dadurch gekennzeichnet, dass** es Mittel (20) zum Sicherstellen einer Nivellierung der Höhe von zwei aufeinander folgenden Platten (1) umfasst.

18. System gemäß Anspruch 17, **dadurch gekennzeichnet, dass** die Mittel (20) durch einen Stift gebildet werden, welcher mit zwei Bohrungen (22) in Eingriff gelangt, welche in den unteren Teil der Flügel (5) der Platten (1) gebohrt sind.

19. System gemäß einem der vorhergehenden Ansprüche, **dadurch gekennzeichnet, dass** jede Platte (1) ausgehend von einem metallischen Blech einer Dicke der Größenordnung von 1,5 bis 2 mm gefertigt ist.

20. System gemäß Anspruch 19, **dadurch gekennzeichnet, dass** jede Platte (1) in Form eines Kastens gefertigt ist, welcher ausgehend von zwei metallischen Blechen gebildet ist, die miteinander durch den unteren (25) und oberen (27) Gurt verbunden sind.

21. Wand zum Zurückhalten von Wasser, insbesondere Schwimmbeckenwand, **dadurch gekennzeichnet, dass** sie ausgehend von einem System wie in einem der vorhergehenden Ansprüche definiert gefertigt ist.
